# FASCICULE DE BREVET EUROPEEN

(11) **EP 1 029 035 B1**
(45) Date de publication et mention de la délivrance du brevet: **28.02.2007**
(21) Numéro de dépôt: 98955664.2
(22) Date de dépôt: 16.11.1998
(51) Int. Cl.: C12N 5/08, A61K 35/55

(54) **PROCEDE POUR INDUIRE LA DIFFERENCIATION OSTEOBLASTIQUE DE CELLULES DE TISSU ADIPEUX EXTRAMEDULLAIRE HUMAIN**
VERFAHREN ZUR INDUZIERUNG DER OSTEOBLASTISCHEN DIFFERENZIERUNG DER ZELLEN DES MENSCHLICHEN EXTRAMEDULLÄREN FETTGEWEBES
METHOD FOR INDUCING OSTEOBLAST DIFFERENTIATION OF HUMAN EXTRAMEDULLARY ADIPOSE TISSUE CELLS

(30) Priorité: 14.11.1997 FR 9714328
(43) Date de publication de la demande: 23.08.2000
(73) Titulaire: BIO HOLDINGS INTERNATIONAL LIMITED, Tortola (VG)
(72) Inventeur: PATAT, Jean-Louis, F-75017 Paris (FR); DARONDEL, Jean, F-14340 Cambremer (FR); OUHAYOUN, Jean-Pierre, F-75006 Paris (FR); LECOEUR, Alain, F-75013 Paris (FR)
(74) Mandataire: Nevant, Marc
(86) Numéro de dépôt international: PCT/FR1998/002438
(87) Numéro de publication internationale: WO 1999/025813

(56) Documents cités:
- WO-A-97/18842
- LECOEUR L ET AL: "In vitro induction of osteogenic differentiation from non -osteogenic mesenchymal cells" BIOMATERIALS, vol. 18, no. 14, juillet 1997, page 989-993 XP004074536
- HAUNER ET AL: "PROMOTING EFFECT OF GLUCOCORTICOIDS ON THE DIFFERENTIATION OF HUMAN ADIPOCYTE PRECURSOR CELLS CULTURED IN A CHEMICALLY DEFINED MEDIUM" JOURNAL OF CLINICAL INVESTIGATION, vol. 84, 1989, pages 1663-1670, XP002073112 cité dans la demande

## Description

L'invention a pour objet un procédé pour obtenir des cellules à phénotype ostéoblastique à partir de cellules présentes dans le tissu adipeux extramédullaire humain. Les cellules obtenues peuvent être utilisées dans la réalisation d'implants osseux.

On sait que pour remédier aux pertes de tissu osseux consécutives à des blessures ou à des opérations chirurgicales, on peut procéder à l'implantation de matériaux de remplacement ou de comblement. Ces matériaux peuvent être des greffons osseux ou des produits artificiels tels que des céramiques poreuses, ou encore des produits naturels tels que le squelette de corail.

La réalisation d'allogreffes comporte notamment des risques de transmission de certaines maladies virales graves. La réalisation d'autogreffes est plus satisfaisante de ce point de vue, mais le prélèvement du greffon nécessite une intervention chirurgicale qui présente des risques de morbidité importants.

Pour ces raisons, on a préconisé l'utilisation d'implants à base de matériaux biocompatibles, éventuellement biodégradables, comme le phosphate tricalcique, l'hydroxyapatite, le plâtre, le corail, les polymères à base de poly(acide lactique), etc. Les matériaux macroporeux sont particulièrement intéressants, car la présence de pores favorise la repousse osseuse.

Depuis quelques années, la recherche s'oriente vers l'utilisation de cellules à potentiel ostéogénique combinées ou non à des biomatériaux ; voir par exemple le brevet français n° 2 679 250, qui envisage la culture d'ostéoblastes sur un support solide tridimensionnel poreux constitué par du squelette de corail en vue d'une implantation chez des patients souffrant d'une perte de substance osseuse, déjà réalisée ou prévisible (lorsqu'une résection chirurgicale est envisagée).

L'intérêt de telles méthodes est évidemment de permettre la réalisation d'implants à l'aide de cellules autologues.

Dans ce domaine, la recherche s'est orientée vers l'utilisation de cellules de moelle osseuse qui sont capables de se différencier en ostéoblastes, notamment sous l'influence de certains facteurs de croissance ayant un effet ostéoinducteur. Il est en effet connu que des cultures de cellules de moelle osseuse sont capables de s'orienter notamment vers le développement de phénotypes ostéoblastiques.

Toutefois, le prélèvement de moelle osseuse présente les mêmes inconvénients que le prélèvement de greffons osseux.

Des études effectuées sur le lapin ont montré que des cellules du compartiment stroma-vasculaire de tissu adipeux extramédullaire sont capables de se différencier en ostéoblastes dans des cultures *in vitro* en présence du facteur ostéoinducteur BMP2 et de dexaméthasone ; voir L. Lecoeur et al., Cellular Engineering; Vol 2, n° 2, 1-7 (1997). La dexaméthasone seule ne permet pas cette différenciation.

On a maintenant découvert qu'au contraire, la culture *in vitro* de certaines cellules de tissu adipeux extramédullaire humain peut conduire à une différenciation ostéoblastique en présence d'un glucocorticoïde seul, par exemple en présence de dexaméthasone seule, c'est-à-dire non associée à un facteur ostéoinducteur. L'utilisation de tels facteurs, qui sont des produits coûteux, n'est pas nécessaire.

Parmi les facteurs ostéoinducteurs, on peut citer le médiateur protéique appelé "Bone Morphogenetic Protein" (BMP), décrit par URIST M.R. et al., P.N.A.S. USA 76 : 1828-1832 (1979). En fait, cette désignation englobe plusieurs facteurs protéiques ostéoinducteurs (de BMP2 à BMP9); voir par exemple YAMAGUCHI et al., Sem.Cell.Biol. 6, 165-173 (1993).

L'invention a donc pour objet un procédé pour induire une différenciation ostéoblastique et/ou pour obtenir des cellules engagées dans cette différenciation, au départ de cellules de tissu adipeux extramédullaire humain, comprenant l'étape consistant à faire incuber pendant un temps suffisant lesdites cellules de départ dans un milieu nutritif liquide permettant le développement desdites cellules, ledit milieu nutritif contenant en solution au moins un glucocorticoïde et étant exempt de facteur adipogénique, et en particulier exempt d'insuline, ainsi que de facteur ostéoinducteur.

Autrement dit, le procédé de l'invention est un procédé dans lequel on cultive.les cellules de départ et dans lequel, pour induire une différenciation ostéoblastique et/ou pour obtenir des cellules engagées dans cette différenciation, on ajoute au milieu de culture au moins un glucocorticoïde.

HAUNER H. et al., J. Clin. Invest. 84 : 1663-1670 (1989) ont décrit la différenciation en adipocytes de cellules de tissu adipeux sous-cutané humain par culture en présence d'insuline et de glucocorticoïdes.

Les cellules utilisées comme cellules de départ dans le procédé de l'invention sont des cellules qui, contrairement aux cellules de moelle osseuse, sont capables de se différencier en adipocytes en présence d'insuline ; voir notamment GREENBERGER J.S., IN VITRO, vol. 15, n°10, 823-828 (1979).

Les cellules qui, selon le procédé de l'invention, s'orientent vers la différenciation ostéoblastique ne sont pas les adipocytes matures, qui sont déjà différenciés. Pour cette raison, on préfère utiliser des cellules de départ choisies dans des ensembles de cellules débarrassés des adipocytes matures. Il s'agit de cellules stroma-vasculaires qui peuvent être obtenues notamment par dissociation du tissu adipeux à l'aide de collagénase, puis élimination des adipocytes matures, par exemple par centrifugation : en effet, les adipocytes matures ont une densité plus faible que les autres cellules présentes dans le tissu adipeux de départ et peuvent donc être éliminées par centrifugation.

En pratique, on peut utiliser comme cellules de départ les cellules qui, par exemple après dissociation du tissu adipeux par une collagénase et élimination des adipocytes matures, sont capables d'adhérer sur des surfaces de polystyrène.

On fait incuber les cellules dans le milieu de culture, dans des conditions standard permettant leur développement, c'est-à-dire non seulement leur survie mais aussi leur prolifération et/ou leur différenciation. Les conditions standard de culture de cellules humaines sont connues : par exemple température de 37°C environ ; atmosphère air-CO₂ 95 : 5 ; pH voisin de la neutralité.

Le milieu de culture utilisé est un milieu nutritif liquide classique contenant les ingrédients nécessaires au développement de cellules de mammifères. Ces ingrédients sont connus. Ce sont principalement des sels minéraux (notamment Na, K, Mg, Ca et éventuellement Cu, Fe, Zn), des acides aminés, des vitamines et des sources de carbone (par exemple glucose). On peut utiliser notamment un milieu nutritif tel que le milieu minimum essentiel MEM de EAGLE, complémenté avec du sérum de veau foetal ou, de préférence, avec du sérum humain autologue.

On peut également utiliser des milieux nutritifs plus élaborés, du type DME (milieu de EAGLE modifié par DOLBECCO), éventuellement en mélange avec le milieu F12 de HAM, avec ou sans sérum, et de préférence en présence de sérum autologue.

Ces milieux de culture peuvent être additionnés de facteurs ostéoinducteurs, comme les facteurs BMP, mais, comme indiqué ci-dessus, ces facteurs ne sont pas nécessaires pour induire la différenciation ostéoblastique des cellules humaines utilisées dans le procédé de l'invention. D'après les résultats obtenus, dans la partie expérimentale ci-après, la présence d'un facteur ostéoinducteur (au moins pour certains d'entre eux) peut même avoir un effet défavorable. On peut donc utiliser des milieux exempts de facteurs ostéoinducteurs.

De préférence, les milieux de culture utilisés sont additionnés de facteurs ostéopromoteurs tel que l'acide ascorbique et les bêta-glycérophosphates (par exemple de sodium ou de calcium).

Par ailleurs, les milieux de culture utilisés dans le procédé de l'invention sont exempts de facteurs adipogéniques, et en particulier exempts d'insuline. On notera à ce propos que les glucocorticoïdes n'ont un effet adipogénique, sur les cellules de la fraction stroma-vasculaire de tissus adipeux extramédullaires, qu'en présence d'insuline ; voir HAUNER et al., article cité ci-dessus.

On peut effectuer la culture dans des boîtes de culture classiques. On peut aussi effectuer la culture sur un support solide tridimensionnel biocompatible immergé dans le milieu de culture liquide.

On peut faire incuber les cellules avec le glucocorticoïde soit dès le début de la culture, juste après l'ensemencement, soit ultérieurement, par exemple après que les cellules sont arrivées à confluence, ou à tout moment entre ces deux événements.

Les glucocorticoïdes qui conviennent sont ceux qui permettent d'obtenir, après incubation des cellules pendant un temps suffisant en présence du glucocorticoïde étudié, des cultures contenant des cellules engagées dans une différenciation ostéoblastique.

Dans la présente demande, on considère que des cellules sont engagées dans une différenciation ostéoblastique lorsqu'elles remplissent au moins les deux premières des conditions suivantes :
- production de phosphatase alcaline de type os-foie-rein,
- production de collagène de type I,
- production d'ostéocalcine.

De telles cellules, qui sont dites aussi "cellules à phénotype ostéoblastique", sont suffisamment engagées dans la différenciation ostéoblastique pour que la poursuite de leur incubation dans le milieu nutritif et/ou leur implantation permette à au moins une partie desdites cellules d'avancer dans la différenciation, jusqu'à la différenciation terminale (avec production d'une matrice extracellulaire minéralisée). La mise en évidence des propriétés caractéristiques de ces cellules (à savoir production de phosphatase alcaline, de collagène de type I, et éventuellement production d'ostéocalcine) peut être effectuée de façon classique, par exemple à l'aide des tests mentionnés dans la partie expérimentale ci-après.

Il convient de rappeler ici que chez les humains, il existe trois isoenzymes de la phosphatase alcaline, codées par trois gènes différents, à savoir :
- une isoenzyme de type os-foie-rein,
- une isoenzyme de type placentaire,
- une isoenzyme de type intestinal.

La phosphatase alcaline de type os-foie-rein est très sensible au levamisole.

La concentration en levamisole entraînant une inhibition de 50% de l'activité de la phosphatase alcaline de type os-foie-rein est de l'ordre de 0,03mM, tandis que pour les autres phosphatases alcalines, l'inhibition de 50% de l'activité n'est obtenue qu'avec des concentrations en levamisole de l'ordre de 1mM pour le type placentaire et de 3mM pour le type intestinal.

Le test d'inhibition par le levamisole permet donc de distinguer sans ambiguïté la phosphatase alcaline de type os-foie-rein. Ce test peut être mis en oeuvre de la façon suivante. On prélève des extraits cellulaires (50 microlitres) et on les fait incuber dans 100 microlitres d'une solution contenant du 2-amino-2-méthyl-1-propanol 1,5mM, de pH 10,3, et 100 microlitres d'une solution de phosphate de para-nitrophénol 14mM, en présence de concentrations croissantes de levamisole, allant de 10⁻⁶M à 10 ⁻⁴M. La réaction est effectuée à 37°C et est arrêtée au bout de 20 minutes par addition de 100 microlitres d'une solution 0,33M de NaOH. La concentration en levamisole produisant une inhibition de 50% peut ainsi être déterminée.

Grâce aux tests décrits dans la présente demande, on peut donc déterminer par des expériences de routine les glucocorticoïdes naturels, ou leurs analogues de synthèse, qui conviennent, ainsi que les concentrations en glucocorticoïde à utiliser et le temps d'incubation suffisant des cellules en présence du glucocorticoïde.

Parmi les glucocorticoïdes, on peut citer notamment la dexaméthasone, l'hydrocortisone, la prednisolone, la méthylprednisolone, la prednisone, la triamcinolone, la corticostérone, la fluocinolone, la cortisone, la bêtaméthasone, etc.

Le temps d'incubation est au moins égal au temps nécessaire pour obtenir des cellules répondant au moins aux deux premières conditions mentionnées ci-dessus. Ce temps est généralement de l'ordre de 15 jours. Pour obtenir des cellules produisant en outre de l'ostéocalcine, la durée du traitement est de l'ordre de 30 jours. Pour obtenir des cellules encore plus avancées dans la différenciation, et produisant une matrice extracellulaire minéralisée, il faut en général de 35 à 50 jours, dans les conditions qui ont été étudiées.

Les concentrations en glucocorticoïde dépendent notamment de la nature du glucocorticoïde. Ces concentrations peuvent être déterminées à l'avance dans chaque cas par de simples expériences de routine. Généralement, on utilise des concentrations de l'ordre de 10⁻⁵ à 10⁻¹⁰ M, en particulier de 10⁻⁶ à 10⁻⁸ M.

On peut mettre en oeuvre le procédé de l'invention en réalisant la culture dans des boîtes de culture classiques ou sur un support solide tridimensionnel biocompatible, poreux ou non poreux, immergé dans un milieu liquide. Le procédé peut également être mis en oeuvre par culture préliminaire dans des boîtes de culture classiques (éventuellement en présence d'un glucocorticoïde), puis transfert des cellules obtenues sur un support solide tridimensionnel immergé dans un milieu de culture liquide.

Avant de s'engager dans la différenciation, les cellules de départ commencent généralement par proliférer, jusqu'à confluence. Lors de cette phase de prolifération, il est possible d'ajouter un glucocorticoïde au milieu de culture, ce qui a en général pour effet d'accélérer le processus de différenciation. Après confluence, lorsqu'elle est observable (cas de cultures en boîtes de culture ou sur support solide tridimensionnel non poreux), ou après un temps de culture prédéterminé (cas de cultures sur un support solide tridimensionnel poreux) commence la phase de différenciation proprement dite pendant laquelle on fait incuber les cellules en présence, obligatoire à ce stade, d'un glucocorticoïde.

Les cellules ayant proliféré, ou en voie de différenciation dans des boîtes de culture peuvent être transférées sur des supports solides tridimensionnels en vue de les faire se multiplier et/ou de poursuivre le processus de différenciation par incubation du support solide dans un milieu nutritif liquide contenant si nécessaire un glucocorticoïde. Les cellules engagées dans la différenciation ostéoblastique, obtenues en boîtes de culture, peuvent être transférées sur un support solide tridimensionnel, par exemple par imprégnation dudit support avec une suspension liquide contenant lesdites cellules. Les supports imprégnés ainsi obtenus peuvent être implantés chez un être humain (notamment chez le donneur du tissu adipeux utilisé comme produit de départ). Ces supports imprégnés peuvent aussi être remis en culture en les immergeant dans un milieu de culture liquide, éventuellement en présence d'un glucocorticoïde (notamment lorsque les cellules transférées contiennent encore des cellules non différenciées), avant d'être finalement implantés.

Le support solide tridimensionnel doit être biocompatible, de façon à pouvoir être implanté chez un humain. Il peut se présenter sous toute forme appropriée telle que cylindres, sphères, plaques, ou pièces de forme quelconque.

Parmi les matériaux pour le support solide tridimensionnel biocompatible, on peut citer notamment le carbonate de calcium, et en particulier l'aragonite, notamment sous la forme de squelette de corail, les céramiques poreuses à base d'alumine, de zircone, de phosphate tricalcique et/ou d'hydroxyapatite, les répliques de squelette de corail obtenues par échange hydrothermal permettant la transformation du carbonate de calcium en hydroxyapatite (brevet français n° 2 223 325), ou encore des céramiques vitreuses apatite-wollastonite, des céramiques vitreuses bioactives telles que les verres Bioglass® (KITSUGI et al., J. Biomed. Mater. Res. 21 : 1255-1271 (1987), etc.

On peut obtenir et/ou cultiver, conformément à l'invention, les cellules engagées dans la transformation ostéoblastique soit à la surface des supports solides tridimensionnels, s'ils ne sont pas poreux, soit à la surface et dans les pores des solides tridimensionnels poreux.

L'utilisation d'un support à base de corail est particulièrement intéressante. On sait en effet que le corail peut constituer une prothèse osseuse progressivement biodégradable, dont la réhabitation par l'os à mesure de la dégradation (voir par exemple le brevet français n° 2 460 657) sera favorisée par les cellules à phénotype ostéoblastique obtenues conformément à la présente invention. La culture de cellules sur un support de corail est décrite notamment dans le brevet français n° 2 679 250 et dans le brevet US correspondant n°5480827.

De préférence, on utilise comme matériau support poreux un matériau ayant des diamètres de pores compris entre 50 et 250µm, avec une porosité comprise généralement entre 20 et 80%. C'est le cas notamment avec le corail des genres Porites, Acropora, Goniopora, Lobophyllia, Symphillia et Millipora.

L'invention a également pour objet l'utilisation d'un glucocorticoïde, dans un milieu de culture liquide permettant le développement en culture de cellules humaines, comme additif destiné à induire la différenciation ostéoblastique sur des cellules de tissu adipeux extramédullaire humain en culture dans ledit milieu. Bien entendu, le milieu de culture est exempt de facteur adipogénique.

L'invention concerne également des cellules engagées dans la différenciation ostéoblastique obtenues selon le procédé décrit précédemment, ces cellules répondant au moins aux deux premières des conditions suivantes:
- production de phosphatase alcaline de type os-foie-rein,
- production de collagène de type I,
- éventuellement production d'ostéocalcine,
- et éventuellement production d'une matrice extracellulaire minéralisée.

Ces cellules peuvent se présenter sous la forme d'une culture tapissant les pores et/ou la surface d'un support solide tridimensionnel biocompatible, comme indiqué précédemment.

Lorsqu'elles sont sous la forme de cellules obtenues dans des boites de culture classiques, elles peuvent être utilisées pour ensemencer de tels supports solides tridimensionnels poreux et biocompatible. On peut également ensemencer de tels supports solides tridimensionnels sur lesquels on a déjà cultivé une première couche de cellules telles que des fibroblastes qui peuvent être d'origine autologue (voir notamment le brevet français n° 2 679 250), cette première couche servant d'assise aux cellules à phénotype ostéoblastique que l'on souhaite obtenir et/ou cultiver.

Les cellules obtenues selon l'invention, en particulier sous la forme d'un support tridimensionnel imprégné à l'aide d'une suspension contenant lesdites cellules, peuvent être utilisées dans la réalisation d'un implant osseux. Pour cela, les supports tridimensionnels poreux biocompatibles chargés de cellules, peuvent être mis en place comme implants ostéoformateurs. Ils peuvent par exemple être implantés comme pièces de remplacement ou de comblement d'un tissu osseux et sont progressivement et colonisés par un tissu osseux néoformé. De tels supports peuvent également être implantés chez un humain, notamment chez le donneur des cellules de tissu extramédullaire adipeux utilisées comme cellules de départ, de préférence après imprégnation du support solide poreux tridimensionnel avec un facteur de croissance ostéoinducteur, dans un site non osseux, par exemple dans un tissu conjonctif, où ils donneront naissance à un tissu osseux utilisable ultérieurement comme matériau d'autogreffe osseuse.

Les cellules engagées dans la différenciation ostéoblastique obtenues conformément à l'invention peuvent donc être utilisées pour obtenir un produit solide tridimensionnel pouvant servir d'implant osseux, comme décrit ci-dessus. Une telle utilisation fait partie de l'invention.

On va maintenant décrire certaines expériences qui ont conduit à la présente invention.

On a prélevé les biopsies de tissu adipeux lors de plasties abdominales pratiquées chez des patients sains, d'âge compris entre 25 et 50 ans.

Les tissus prélevés sont émincés en petits fragments et digérés pendant 90 minutes avec de la collagénase (2mg/ml) dans du tampon Krebs Ringer à 37°C. On filtre sur un tamis ayant des dimensions de pores de 100µm, on recueille le filtrat et on le centrifuge à 1000 tours par minute pendant 5min. Le surnageant est éliminé et le culot cellulaire est lavé à plusieurs reprises dans le tampon Krebs Ringer. On répète plusieurs fois les étapes de lavage et de centrifugation en éliminant à chaque fois le surnageant contenant des adipocytes matures. Les cellules obtenues sont mises en suspension dans le milieu de culture DME/F12 (SIGMA CHEMICAL Co, St Louis, Mo, USA ; référence D-6905) contenant 10% de sérum de veau foetal, 100µg/ml de streptomycine et 100U/ml de pénicilline.

On ensemence des boîtes de culture en polystyrène ("Tissue culture flasks" commercialisés par BECTON-DICKINSON sous les références 3013, 3028 et 3084) avec la suspension cellulaire ainsi obtenue, et on fait incuber à 37°C sous atmosphère d'air humide additionné de 5% de CO₂.

Au bout de 12 heures, on élimine les cellules non adhérentes par lavage avec du tampon PBS.

On cultive ensuite les cellules adhérentes dans le milieu de culture mentionné ci-dessus, en changeant le milieu tous les 3 jours, jusqu'à confluence.

Les cellules sont ensuite trypsinisées et réensemencées dans des boîtes multipuits à raison de 2.10⁴ cellules/ml, dans un milieu DME/F12 contenant 10% de sérum de veau foetal, complémenté avec 50µg/ml d'acide ascorbique et avec du bêta-glycérophosphate disodique à une concentration de 10mM, et contenant de la streptomycine et de la pénicilline aux concentrations déjà indiquées précédemment. On fait incuber à 37°C.

A confluence, les cellules reçoivent l'un des traitements suivants :
- Traitement n° 1 : addition de rhBMP2 (BMP2 recombinante humaine fournie par GENETIC INSTITUTE, Cambridge, Massachusetts, USA) à raison de 200ng/ml,
- Traitement n° 2 : addition de dexaméthasone jusqu'à une concentration de 10⁻⁷M
- Traitement n° 3 : addition de rhBMP2 200ng/ml + dexaméthasone 10⁻⁷M,
- Témoins non traités : incubation dans le milieu de culture (exempt de rhBMP2 et exempt de dexaméthasone).

Les cultures sont poursuivies pendant 30 jours ou davantage, en renouvelant les milieux de culture ou de traitement tous les 3 jours.

Des échantillons des cultures traitées et non traitées ont été analysés pour déterminer l'activité de phosphatase alcaline, l'expression de collagène de type I et l'ostéocalcine. En outre, la minéralisation éventuelle de la matrice extracellulaire a été recherchée.

La détermination de l'activité de phosphatase alcaline a été effectuée en utilisant le phosphate de para-nitrophénol comme substrat, selon la technique décrite par L. Lecoeur et J. P. Ouhayoun, Biomaterials 18, 989-993 (1997). La quantité de para-nitrophénol formé par hydrolyse du substrat est déterminée en mesurant l'absorbance à 410nm, qui est convertie en nanomoles d'enzyme à l'aide d'une courbe étalon établie à partir de concentrations connues de para-nitrophénol.

On a également effectué un test permettant de détecter *in situ* l'activité de phosphatase alcaline sur les cultures cellulaires fixées à l'aide de formaldéhyde, en utilisant un kit pour la détection histochimique semi-quantitative de la phosphatase alcaline (kit commercialisé par SIGMA CHEMICAL Co., référence 86R). L'activité de phosphatase alcaline est visualisée sur le tapis cellulaire par une coloration rougeâtre.

Par ailleurs, les essais d'inhibition par le levamisole ont montré que la phosphatase alcaline produite est de type os-foie-rein.

Sur des cellules fixées au formaldéhyde, on a également recherché la présence de collagènes (de type I et de type II) et d'ostéocalcine, ainsi que la présence d'une minéralisation éventuelle.

La synthèse de collagène a été analysée, après 30 jours de traitement, à l'aide d'anticorps (IgG) anti-collagène commercialisés par SOUTHERN BIOTECHNOLOGY ASSOCIATES, USA.

De même, la synthèse d'ostéocalcine a été recherchée à l'aide d'un anticorps (IgG) anti-ostéocalcine commercialisé par BIOMEDICAL TECHNOLOGIES INC., USA.

Les déterminations avec les anticorps ont été réalisés en immunofluorescence, en utilisant pour la révélation un anticorps anti-IgG couplé à la fluorescéine.

La recherche de la minéralisation éventuelle de la matrice extracellulaire a été effectuée en utilisant le test de coloration de von Kossa, mis en oeuvre selon la technique décrite par Cheng et al., Endocrinology 134 : 277-285 (1994).

Les résultats observés sont décrits ci-après :

### Activité de phosphatase alcaline

Les cultures traitées avec rhBMP2 seule à la concentration de 200ng/ml ne présentent pas une forte activité de phosphatase alcaline. Dans la plupart des cas, cette activité est inférieure ou égale à celle présente dans les cultures témoins, même après 35 jours d'incubation.

Dans tous les cas, les activités de phosphatase alcaline les plus élevées ont été obtenues dans les cultures traitées ayant subi le traitement n° 2 et les cultures ayant subi le traitement n° 3. Avec le traitement n° 2, dans la quasi-totalité des cas ou a observé, une activité au moins égale à 200 nanomoles de para-nitrophénol/30min/puits, et pouvant atteindre 700 nanomoles/30min/puits.

Avec le test cytoenzymatique, la recherche de l'activité de phosphatase alcaline décelée sur les cultures cellulaires fixées par le formol, après 30 jours de traitement, a donné les résultats suivants : les cultures ayant subi le traitement n° 2 ou le traitement n° 3 ont présenté une majorité de cellules ayant une activité de phosphatase alcaline positive. Les cellules présentant cette activité ont une morphologie étoilée. Les cultures ayant subi le traitement n° 1 ne présentent qu'une faible proportion de cellules positives à la phosphatase alcaline, et ces cellules ont une morphologie fusiforme. Dans les cultures témoins, les cellules exprimant l'activité de phosphatase alcaline sont rares.

### Collagène de type I

Les cultures effectuées, pendant 30 jours, avec le milieu de culture seul (témoins non traités) ne présentent qu'une faible réaction avec l'anticorps anti-collagène de type I. Les cultures ayant subi le traitement n° 1 ont également peu réagi avec cet anticorps. Les cultures ayant subi le traitement n° 2 ont développé des structures nodulaires fortement fluorescentes. Le traitement n° 3 n'a pas induit de telles structures et seule une faible fluorescence a été localisée au niveau de quelques regroupements cellulaires.

### Collagène de type II

Au bout de 30 jours, les cultures traitées n'ont présenté qu'un faible marquage fluorescent, tout comme les cultures témoins.

### Ostéocalcine

Les cultures témoins, ou les cultures ayant subi le traitement n° 1, ont très faiblement réagi avec l'anticorps anti-ostéocalcine. Les cultures ayant subi le traitement n° 3 ont présenté certaines zones avec un marquage légèrement plus intense. La réaction la plus forte a été obtenue avec les cultures traitées à la dexaméthasone seule. Les structures nodulaires induites dans ces cultures ont présenté un très fort marquage fluorescent. En revanche, le tapis cellulaire environnant n'a pas été marqué et la synthèse d'ostéocalcine semble restreinte à ces structures nodulaires.

### Examen des cellules par microscopie électronique à balayage

Les cellules témoins incubées avec le milieu de culture seul présentent un aspect homogène, avec une morphologie assez massive. Les cellules ayant subi le traitement n° 1 présentent une morphologie plus allongées. Les cultures ayant subi le traitement n° 3 ont développé des cellules avec un aspect plus hétérogène et une morphologie irrégulière. Sur les cultures ayant subi le traitement n° 2, la microscopie électronique à balayage a mis en évidence la présence de structures nodulaires.

### Minéralisation in vitro

Au bout de 30 jours, les cultures traitées ou non traitées n'avaient pas développé de matrice extracellulaire minéralisée.

Au bout de 45 jours, sur les cellules non traitées, on n'observe que quelques très rares points de minéralisation. Avec les cellules ayant subi le traitement n° 1 ou le traitement n° 3, aucun point de minéralisation n'a pu être détecté. Sur les cultures ayant subi le traitement n° 2, de nombreux foyers de minéralisation ont été observés, y compris des nodules minéralisés.

## Revendications

1. Procédé pour induire une différenciation ostéoblastique et/ou pour obtenir des cellules engagées dans la différenciation ostéoblastique, au départ de cellules de tissu adipeux extramédullaire, dans lequel on fait incuber pendant un temps suffisant lesdites cellules de départ dans un milieu nutritif liquide permettant le développement en culture desdites cellules,
ledit milieu nutritif contenant au moins un glucocorticoïde, **caractérisé en ce que** ledit milieu nutritif est exempt de facteur adipogénique et de facteur ostéoinducteur pour engager lesdites cellules dans la différenciation ostéoblastique et,
**en ce que** lesdites cellules de départ étant des cellules humaines.

2. Procédé selon la revendication 1, dans lequel ledit milieu de culture est exempt d'insuline.

3. Procédé selon la revendication 1 ou 2, dans lequel lesdites cellules de départ sont débarrassées des adipocytes matures et/ou sont capables d'adhérer sur une surface de polystyrène.

4. Procédé selon l'une quelconque des revendications 1 à 3, dans lequel on fait incuber les cellules pendant un temps suffisant pour obtenir une culture présentant au moins les deux premières des caractéristiques suivantes :
- production de phosphatase alcaline de type os-foie-rein,
- production de collagène de type I,
- production d'ostéocalcine,
- production d'une matrice extracellulaire minéralisée.

5. Procédé selon l'une quelconque des revendications 1 à 4, dans lequel le glucocorticoïde est choisi parmi la dexaméthasone, l'hydrocortisone, la prednisolone, la méthylprednisolone, la prednisone, la triamcinolone, la corticostérone, la fluocinolone, la cortisone et la bêtaméthasone.

6. Procédé selon l'une quelconque des revendications 1 à 5, dans lequel on imprègne un support solide tridimensionnel biocompatible avec une suspension des cellules de départ et on effectue ladite incubation en immergeant le support ainsi imprégné dans ledit milieu de culture.

7. Procédé selon l'une quelconque des revendications 1 à 6, dans lequel on recueille des cellules engagées dans la différenciation ostéoblastique obtenues, puis on imprègne un support solide tridimensionnel biocompatible avec une suspension contenant les cellules recueillies, et, si désiré, on cultive lesdites cellules recueillies.

8. Procédé selon la revendication 6 ou 7, dans lequel le support solide tridimensionnel est réalisé en au moins un matériau choisi parmi le carbonate de calcium, l'hydroxyapatite, le carbonate de calcium recouvert en surface d'hydroxyapatite, et des céramiques.

9. Utilisation, en tant qu'additif à un milieu nutritif liquide permettant le développement en culture de cellules humaines, d'au moins un glucocorticoïde comme seul additif nécessaire pour induire la transformation ostéoblastique de cellules de tissu adipeux extramédulaire humain cultivées dans ledit milieu, lequel milieu de culture est exempt de protéines à effet ostéoinducteur et de facteur adipogénique.

10. Procédé de préparation d'un implant qui comprend les étapes suivantes :
a) obtention de cellules engagées dans la différentiation ostéoblastique selon le procédé de l'une des revendications 1 à 8 ;
b) réalisation de l'implant osseux à partir des cellules ainsi obtenues.

## Claims

1. A method for inducing osteoblast differentiation and/or for obtaining cells engaged in osteoblast differentiation, starting from extramedullary adipose tissue cells, in which the said starting cells are incubated in a liquid nutrient medium for a period of time sufficient to enable said cells to develop in culture, said nutrient medium containing at least one glucocorticoid, **characterized in that** said nutrient medium is free from adipogenic factors and osteo-inducing factors in order to engage the said cells in osteoblast differentiation and, **in that** the said starting cells are human cells.

2. The method according to claim 1, in which said culture medium is free from insulin.

3. The method according to claim 1 or 2, in which said starting cells are separated from mature adipocytes, and/or are capable of adhering to a polystyrene surface.

4. The method according to any one of claims 1 to 3, in which the cells are incubated for a period of time sufficient to obtain a culture having at least the first two of the following characteristics:
- production of alkaline phosphatase of bone-liver-kidney type;
- production of type I collagen;
- production of osteocalcine;
- production of a mineralized extracellular matrix.

5. The method according to any one of claims 1 to 4, in which the glucocorticoid is selected from dexamethasone, hydrocortisone, prednisolone, methylprednisolone, prednisone, triamcinolone, corticosterone, fluocinolone, cortisone, and betamethasone.

6. The method according to any one of claims 1 to 5, in which a biocompatible three-dimensional solid support is impregnated with a suspension of the starting cells and said incubation is performed by immersing the support impregnated in this way in said culture medium.

7. The method according to any one of claims 1 to 6, in which resulting cells engaged in osteoblast differentiation are recovered, and then a biocompatible three-dimensional solid support is impregnated with a suspension containing the recovered cells, and if so desired, said recovered cells are cultured.

8. The method according to claim 6 or 7, in which the three-dimensional solid support is made out of at least one material selected from calcium carbonate, hydroxyapatite, calcium carbonate surface-coated with hydroxyapatite, and ceramics.

9. The use, as an additive in a liquid nutrient medium enabling human cells to develop in culture, of at least one glucocorticoid as the only additive required to induce osteoblast transformation of human extramedullary adipose tissue cells cultured in said medium, said culture medium being free from proteins having an osteo-inducing effect and adipogenic factors.

10. A preparation process of an implant comprising the following steps:
a) obtaining cells engaged in osteoblast differentiation according to the method of one of claims 1 to 8;
b) Production of a bone implant from the resulting cells.

## Patentansprüche

1. Verfahren zur Induzierung einer osteoblastischen Differenzierung und/oder zum Erhalt von bei der osteoblastischen Differenzierung eingesetzten Zellen aus extramedullären Fettzellgewebezellen, bei dem man für eine ausreichende Zeit die Ausgangszellen in einem flüssigen Nährmedium inkubieren läßt, das die Entwicklung der Zellen in Kultur erlaubt,
wobei das Nährmedium wenigstens ein Glucocorticoid enthält,
**dadurch gekennzeichnet, daß** das Nährmedium frei von adipogenem Faktor und Osteoinduktionsfaktor ist, um die Zellen bei der Osteoblastendifferenzierung wirken zu lassen, und
**dadurch**, daß die Ausgangszellen Humanzellen sind.

2. Verfahren nach Anspruch 1, bei dem das Kulturmedium frei von Insulin ist.

3. Verfahren nach Anspruch 1 oder 2, **dadurch gekennzeichnet, daß** die Ausgangszellen von maturierten Adipocyten befreit sind und/oder in der Lage sind, sich auf einer Polystyroloberfläche anzuheften.

4. Verfahren nach einem der Ansprüche 1 bis 3, bei dem man die Zellen für eine ausreichende Zeit inkubieren läßt, um eine Kultur zu erhalten, die wenigstens die beiden ersten der folgenden Merkmale aufweist:
- Herstellung von alkaliner Phosphatase vom Typ Knochen-Leber-Niere,
- Herstellung von Typ-I-Kollagen
- Herstellung von Osteocalcin,
- Herstellung von einer mineralisierten extrazellulären Matrix.

5. Verfahren nach einem der Ansprüche 1 bis 4, bei dem das Glucocorticoid gewählt ist unter Dexamethason, Hydrokortison, Prednisolon, Methylprednisolon, Prednison, Triamcinolon, Corticosteron, Fluocinolon, Kortison und Betamethason.

6. Verfahren nach einem der Ansprüche 1 bis 5, bei dem man einen festen dreidimensionalen biokompatiblen Träger mit einer Suspension der Ausgangszellen imprägniert und man die Inkubation durchführt, indem man den so imprägnierten Träger in Kulturmedium eintaucht.

7. Verfahren nach einem der Ansprüche 1 bis 6, bei dem man erhaltene, in der Osteoblastendifferenzierung eingesetzte Zellen aufnimmt und dann einen festen dreidimensionalen biokompatiblen Träger mit einer Suspension tränkt, die die aufgenommen Zellen enthält und, wenn gewünscht, die aufgenommenen Zellen kultiviert.

8. Verfahren nach Anspruch 6 oder 7, bei dem der feste dreidimensionale Träger wenigstens teilweise aus einem Material ausgeführt ist, das gewählt ist unter Kalziumcarbonat, Hydroxyapatit, Kalziumcarbonat, das auf der Oberfläche mit Hydroxyapatit bedeckt ist, und Keramiken.

9. Verwendung als Zusatz zu einem flüssigen Nährmedium, das die Entwicklung von Humanzellen in Kultur erlaubt, von wenigstens einem Glucocorticoid als einzigem notwendigen Zusatz, um die osteoblastische Transformation von extramedullären Humanfettgewebezellen zu induzieren, die in dem Medium kultiviert sind, welches Kulturmedium frei von Proteinen mit Osteoinduktionswirkung und von adipogenem Faktor ist.

10. Verfahren zur Herstellung eines Implantats, umfassend die folgenden Schritte:
a) Erhalt von Zellen, die bei der osteoblastischen Differenzierung wirken, gemäß dem Verfahren der Ansprüche 1 bis 8;
b) Ausführung des Knochenimplantates aus den so erhaltenen Zellen.
